Europäisches Patentamt

European Patent Office

Office européen des brevets

(11) Publication number: **0 304 871**
**A2**

## (12) EUROPEAN PATENT APPLICATION

(21) Application number: 88113708.7

(22) Date of filing: 23.08.88

(51) Int. Cl.4: **C12Q 1/00 , G01N 33/52 , G01N 33/543 , //A61B10/00**

(30) Priority: 25.08.87 US 89472

(43) Date of publication of application:
01.03.89 Bulletin 89/09

(84) Designated Contracting States:
AT BE CH DE ES FR GB GR LI LU NL SE

(71) Applicant: DENTSPLY MANAGEMENT CORP.
570 West College Avenue
York Pennsylvania 17405(US)

(72) Inventor: Jefferies, Steven R.
715 North Shore Drive
Milford, DE 19963(US)
Inventor: Kopunek, Thomas V.
505 Railroad Avenue
Lewes, DE 19958(US)
Inventor: Dougherty, Emery W.
451 Hunting Park Lane
York, PA 17402(US)
Inventor: Heyde, John B.
940 Club House Road
York, PA 17403(US)
Inventor: Chadwick, David E.
3520 Pebble Ridge Drive
York, PA 17402(US)

(74) Representative: Wächtershäuser, Günter, Dr.
Tal 29
D-8000 München 2(DE)

(54) **Dental diagnostic device and method.**

(57) An apparatus for use in dental diagnosis of a root canal and of periodontal pockets is provided. The apparatus comprises a device for absorbing fluids in the oral cavity and a handle for carrying the device to the oral cavity without contaminating the device by touching it with the fingers. The device of the invention comprises an absorbent material, adapted to be inserted into a root canal or a periodontal pocket, which has a substrate absorbed thereon which reacts with enzymes which are indicative of disease in the oral cavity. A detectable change in fluorescence or color in the substrate indicates the presence of the enzymes. A method of using the device of the invention to detect the presence of bacteria or disease in the oral cavity is also provided.

## DENTAL DIAGNOSTIC DEVICE AND METHOD

### BACKGROUND

This application is a continuation-in-part of U.S. Serial No. 089,472 filed August 25, 1987.

The present invention relates to an apparatus and procedure by which diagnosis of dental infections and diseases of the oral cavity can be made.

The cost efficiency of any antimicrobial treatment, for example antibacterial treatment in dentistry, whether preventative or therapeutic, increases to the extent that it can be found on individuals who are at risk of dental decay or periodontal disease. With the realization that Streptococcus mutans and lactobacilli are primary etiologic agents in dental decay, and spirochetes, Bacteroides gingivalis, A actinomycetemcomitans and others are prime etioligic agents in periodontal disease, those individuals with infections of such bacteria can be identified by testing for the presence of such bacteria, and treating them to prevent the disease or to cure the infections. The basis for general techniques for such testing are described by, among others, Robert E. Smith in "Contributions of Histochemistry of the Development of the Proteolytic Enzyme Detection System in Diagnostic Medicine" Journal of Histochemistry and Cytochemistry 33,199,1983 and Dr. Walter J. Loesche, "The Identification of Bacteria Associated with Periodontal Disease and Dental Caries by Enzymatic Methods", Oral Microbiol Immunol 1986; 1:65-70. Such testing is based, in general, on the discovery that such pathogenic bacteria produce trypsin like enzymes that may be detected and measured by means known in the art for detecting trypsin.

Also, dental and medical procedures deal with the elimination of infection,e.g. dental caries, and the restoration of the organ to function after an infection is removed. The treatment employed may or may not be effective. In many cases the only evidence that an infection is overcome is the determination of the future progress of the disease, particularly the lack of signs of acute recurrence.

Therefore, there is a need in dentistry both to identify those patients who are in need of treatment and to determine when treatment has reduced the effects of infection without waiting for the signs of recurrence. For example, when a tooth has become infected with microorganisms its natural defense mechanisms may fail, the pulp may lyse, pressure from the degradative by-products may strangle the tooth and necrosis may result. One treatment is to extirpate the living pulp, and sterilize the tooth as effectively as possible by the application of antiseptic or antimicrobial irrigants. This is a very old and useful procedure. To assess the completeness of the antimicrobial treatments, the intercanal serum or exudate may be cultured using suitable microbiological growth media. When the culture is negative after sampling, the root canal may be filled with a sealer to block further infection by microorganisms. This procedure is lengthy and requires special skills.

Similarly, the sulcus of the tooth, the space between the tooth and the mucosa, may become infected. As the effects of the infection proceed the soft tissue may withdraw from the tooth and/or become inflamed and a periodontal pocket may form that becomes a breeding ground for further growth of infectious bacteria. The syndrome has been called periodontal disease which is now well established as being bacterial in origin. Depending on complex interactions between the host's defense mechanisms, the precise infection, and the availability of appropriate nutrition and growth conditions for the bacteria, the bacterial infection may cause continual irritation, lysis of the soft tissue and periodontal membrane, and eventually loss of the supporting bone. One method of diagnosis is to remove scrapings from the tooth within the subgingival pocket and, as in the case of endodontic infection, culture the scrapings to determine the type and concentration of infecting bacteria.

In both endodontic and periodontal culturing techniques much depends on the aquisition of a suitable sample, its careful transfer and growth in vitro, and subsequent identificiation of pure bacterial cultures. These procedures require a considerable amount of skill and experience, selection or development of correct bacterial nutrients, a properly equipped laboratory, and a substantial amount of time to complete the assays.

Thus it is an objective of this invention to provide means for the rapid assay of infectious conditions by the dentist in his operatory, using skills familiar to the dentist that have already been developed for the treatment of teeth and dental diseases. More specifically, in a preferred embodiment of the invention, the presence of enzymes, for example, are determined by their reaction with a substrate fixed within an absorptive support, such as a support of the type that is currently used for sampling intercanal or sulcular serum or exudate. The reaction of the enzyme with the substrate is identified by a color indicator, either visual or flourescent, or another indicator, for example a radiographic indicator, which is stoichiometrically associated with the reaction and its extent.

It is known in the art to insert flat strips of absorbent paper impregnated with a lead acetate substrate into periodontal pockets to detect the presence of $H_2S$ as is described by Horowitz et al, in "Hydrogen Sulfide Production in the Periodontal Environment" Journal of Periodontology 7 (44) July 1973, pp 390-395.

The present invention may use measurements of enzymes that are associated with specific bacterial infections, or any of a cascade of enzymes that mediate and control the immediate host response or the immunologic response by the host upon stimulus from an outside source. Enzymes that may be liberated that have specific functions in the destruction of the tissues from whatever cause are, e.g. collagenase, elastase and other proteolytic enzymes. Accordingly, these enzymes and substrates specific for them may also be utilized in this invention.

Similarly, antibodies may be linked with enzymes absorbed within the solid support in a diagnostic technique known as Enzyme Linked Immunosorbent Assay. The assay method may be used to rapidly identify enzymatic and immunologic components of the serum or exudate in conjunction with the teachings of this invention. Other applications will become apparent from the following specification and claims.

## SUMMARY OF THE INVENTION

A device for detecting a disease state characterized by the presence of enzymes, antibodies, antigens or antihistamines indicating the presence of bacteria, tissue regeneration or tissue deterioration in the mouth is provided. The device comprises an absorbent material having a dimension such that it can be inserted into small recesses or pockets in the mouth. The absorbent material of the device has a substrate absorbed thereon which is adapted to react with biological materials that can be associated with the presence of bacteria or with tissue regeneration or with tissue deterioration. The device is adapted to be carried to the mouth by means of a forceps or handle in order to avoid contamination by being touched by the practitioner. The substrate absorbed on the device, when it reacts with a specific biological material, will change color, or alternatively, will release chemicals that react with an indicator to cause a detectable and measurable change in color or fluorescence, indicating the degree and/or nature of the infection.

A method for detecting conditions relating to the presence of bacteria, tissue regeneration or tissue deterioration in the mouth is also provided. The method comprises the steps of absorbing a substrate adapted to react chemically with biological materials that can be associated with the presence of bacteria, tissue regeneration or tissue deterioration onto an absorbent material which is dimensioned to fit into small recesses such as root canals and periodontal pockets in the mouth, inserting the absorbent material with the substrate into a small recess in the mouth and absorbing biological material from the recess onto the absorbent material, allowing the biological material to react with the substrate for a time sufficient for a measurable chemical reaction between the biological material and the substrate to take place, and measuring the nature and/or the extent of the chemical reaction to establish the nature and/or concentration of the reactive component in the biological material. A number of specific substrates that are reactive with a number of specific enzymes may be used in the method of the invention.

A handle for manipulating the device without touching the device with the hands, in order to avoid contamination of the device is also provided.

The present invention in one of its aspects provides for the analysis of the disease state of the mouth. The most preferred aspect is the detection of the presence of enzymes that are associated with bacterial infections and the resulting tissue destruction or host response. However, enzymes associated with tissue repair are another important aspect of the invention. The invention can also be applied to detecting the presence of antigens and antibodies and other similar uses. Thus, by the detection of oral disease it is meant to include the detection of progressive healing state of the mouth as well as the presence of bacteria and the resulting tissue deterioration in the mouth.

In an embodiment of the invention, a known amount of biological fluid is collected and transferred to a substrate, with or without a support, to obtain a quantitative determination of the concentration of disease indicating active materials in oral fluid.

The invention includes aspects of placing a chemical substrate into the mouth which reacts with biological materials present in the mouth that are associated with the diseased state. The reaction may be immediately observable or readable or may require further steps in procedure in the mouth or outside of the mouth to provide a readable indication of the reaction. In a preferred aspect, a method of detecting a disease state in the mouth is provided that includes inserting a support comprising an absorbent material that has a substrate absorbed thereon that reacts with biological material that can be associated with a disease state in the mouth, allowing the biological material to react with the substrate, and evaluating the reaction.

In another preferred aspect a device for detecting the disease state in the mouth is provided, which provides for obtaining samples of oral fluid only.

A disease state in the mouth as used herein is defined as a state caused by microbial infection, for example bacterial infection which manifests itself in deterioration of the tissue of the mouth such as gingivitis or similar periodontal disease and dental caries. In another embodiment, also encompassed by "disease state" is sampling in the mouth for the detection of whole body infections, such as viral infections. The presence of the disease state can be detected by measuring the presence of enzymes associated with bacteria, with enzymes associated with tissue deterioration caused by the bacteria, tissue regeneration or by the detection of antihistamines or antibodies.

As is apparent, the device and method of the invention can also be used to characterize tissue destruction caused by other than bacteria, for example necrosis.

The device and method of the invention provide a simple, time effective means of determining the presence or the nature and degree of oral disease in the mouth. Since the biological material whose concentration is measured to determine the presence of oral disease reacts directly with a substrate on the device used to collect the material, the time consuming and sensitive steps of transferring and/ or culturing the biological material prior to measuring the concentration of the material is avoided. The reaction of the substrate with the biological material may be immediate and is often complete within 40 minutes, and can be incorporated easily into the normal routine of office procedures.

## BRIEF DESCRIPTION OF THE DRAWINGS

Fig. 1 illustrates a handle used for carrying the device of the invention to the oral cavity.

Fig. 2 illustrates a device of the invention.

Fig. 3 illustrates a top view of a kit which can be used for holding devices of the invention for sale and for incubation after biological fluid is collected.

Fig. 4 illustrates a side view of a kit for holding devices of the invention.

Fig. 5 illustrates a color coding strip used to evaluate reactivity of the substrate with biological materials.

Fig. 6 illustrates a kit similar to Fig. 3 used for paper strips.

Fig. 7 is a cross section along line 7-7 of a paper strip in the kit of Fig. 6.

Fig. 8 is a test slide used for reacting substrate impregnated disc with biological fluids.

## DETAILED DESCRIPTION OF THE ILLUSTRATED EMBODIMENTS

Examples of proteolytic bacteria that produce trypsin-like enzymes, and may be implicated in periodontal disease are Bacteroides gingivalis, Bacteroides intermedius, Trepaneoma loesche, A-actinomycetemcomitans, Trepaneoma-denticola, and Bacteroides forsythus. These pathogens are able to hydrolyse various synthetic substrates of trypsin, which fact can be used to identify the presence of these bacteria.

Those skilled in the art will recognize that the detection of any neutral proteolytic enzyme produced by the above or similar bacteria which are indicative of periodontal disease, such as elastase, Cathepsin G or bacterial collagenase type enzymes, may be used in the method of the invention.

Substrates which may be used to detect neutral proteolytic enzymes are

(a) Z-arg-x
(b) Z-ala-ala-x
(c) Z-arg-arg-x
(d) Z-arg-arg-arg-x
(e) Z-gly-arg-x
(f) Z-gly-gly-arg-x
(g) Z-arg-gly-phe-phe-leu-x
(h) Z-ala-ala-ala-x
(i) Z-ala-ala-pro-val-x

(j) Z-ala-ala-pro-ala-x

(k) Z-pro-leu-gly-ile-ala-gly-arg-x

(l) Z-ala-ala-pro-phe-x

(m) Z-gly-pro-leu-gly-pro-x

(n) Z-ala-pro-x

Where Z represents an inert carrier for the protein moiety of the substrate such as MeoSuc, Suc or benzyloxycarbonyl; arg = arginine, ala = alanine, gly = glycine, phe = phenylalanine, leu = leucine, pro = proline, ile = isoleucine, Suc = succinyl, MeoSuc = methoxysuccinyl, val = valine and x is selected from the group comprising 7-amino-4-trifluoromethyl coumarin (AFC), amino trifluoro quinoline (AFQ), 4-methoxy-2-napthylamide (MNA), 7-amino-4-methyl coumarin (AMC), 5-aminoisophthallic acid dimethyl ester (AIE).

In particular, a number of amino acid esters such as a number of similar napthylamide and coumarin derivatives have been found to be useful as such substrates.

An example of the many enzymes produced by the host system in response to tissue destruction caused by bacterial infection are the phosphatases. Acid phosphatase is released by the host in the resorption of damaged tissue, and alkaline phosphatase is released by the host during repair of damaged tissue. Accordingly, the device of the invention can be used in the method of the invention to measure, for example, the concentration of the phosphatase enzymes in the damaged area.

It is known in the art that polyhydric alcohols (such as glucose), and substituted aromatic hydroxy compounds are useful as substrates for detecting the presence of and the concentration of the phosphatases. Examples of such substrates are x-1-glucose-6-phosphate, phenylphosphate, p-nitrophenyl-phosphate, phenolphthalein mono or di phosphate, and $\beta$-glycerolphosphate. The detection of the aromatic hydroxy leaving group of the substrate is particularly adapted to the present invention since phenol, for example, may be easily detected when reacted with a diazo reagent. 4-aminoantipyrine is particularly adapted for use as an indicator in the present invention since detectable results can be obtained in about 15 minutes. The detection of nitrobenzene, when using, for example, p-nitrophenylphosphate as a substrate, is also useful in the present invention because the reaction of the substrate is linear with time and there is no time delay; and the substrate goes from colorless to yellow without the addition of any other reagents.

In general, the presence of a reactive species is detected by observing a distinctive change in color or fluorescence. For the purpose of this application, "color change" represents a visible color change, a change in fluorescence color or intensity, or any similar change in wavelength of absortive radiation or any similar detecting means known in the art.

Other such substrates and detecting systems using the same or other enzymes will be apparent to those skilled in the art.

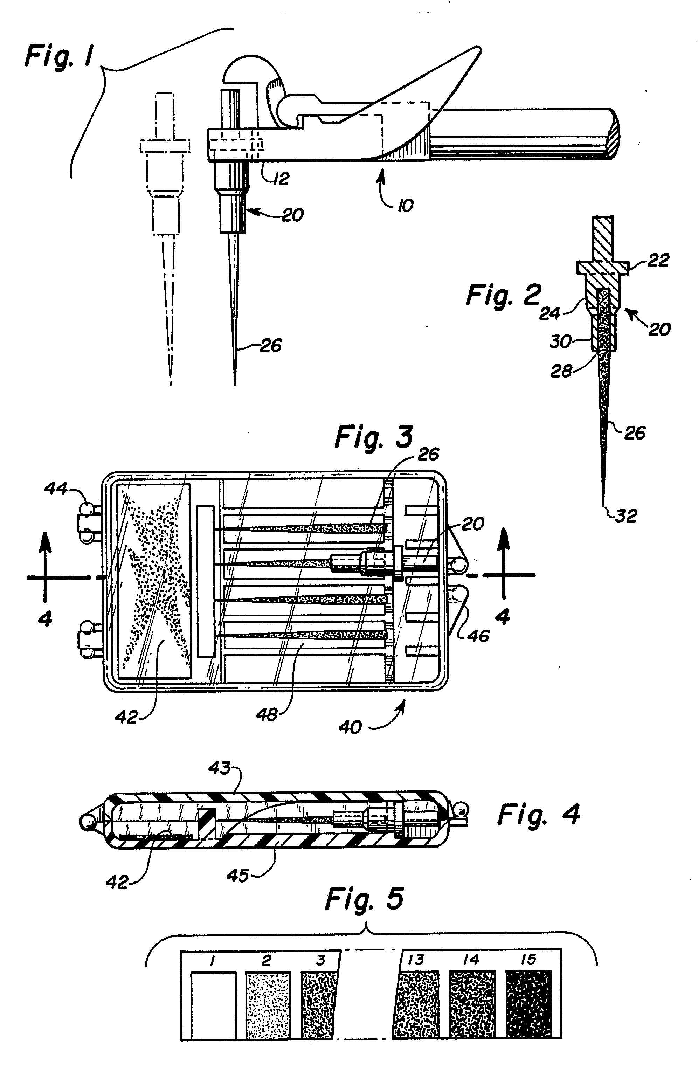

With reference now to Figs.1 and 2 a handle 10 similar to that described in copending application S.N. 035,574 filed April 7, 1987, the contents of which are incorporated herein by reference, is utilized to carry the device 20 of the invention to the oral cavity. The handle 10 has a jaw 12 which is adapted to fit on and hold collar 22 of device 20. Using handle 10 to hold and carry device 20 makes it possible to provide a sterile device 20 that can be stored and manipulated without being touched by the fingers and contaminated by enzymes or bacteria that may be present on the fingers.

Those skilled in the art will recognize that other, simpler means, such as forceps, can be used to carry the reactive portion of the device (cone shaped paper point with absorbed substrate 26) of the invention to easy to reach locations in the mouth. The handle, however, may be provided with means to assure a good hold on the absorbent material (cone) 26, and such a handle may have a shape that makes easy access of the device to hard to reach areas of the mouth.

It will be recognized by those skilled in the art that absorbent material in other forms can be used as a support for a substrate to be used in the same manner as described presently for paper points and the terms ""support" and "absorbent material" are intended to embrace the absorbent material in any such device.

The device 20 comprises an upper body portion 24 for holding absorbent material 26 therein. In the illustrated embodiment, body portion 24 comprises a hollow cylinder having an opening 28 in at least end 30 in upper body portion 24, said opening being adapted to hold absorbent material 26 so that the absorbent material is joined with or to the upper body portion 24. The collar 22 serves as a releasable element for holding device 20 in the jaws 12 of handle 10. The releasable element may be seen to extend perpendicularly to the projection of the absorbent material (cone) 26.

Absorbent material 26 may be held in opening 28 using an adhesive or any similar means known to those skilled in the art. Absorbent material 26 will preferably have a tapered configuration having its widest point at end 30 of body portion 24, and tapering down to a relatively fine point at end 32 of said absorbent material 26.

Absorbent material 26 will have a length that provides for easy access in the mouth and preferably will be about 10-25 mm long, and will have a diameter at its tip in the range of about 0.25 mm - 1.0 mm. More preferably the absorbent material will be about 15 mm - 20 mm long and will have a diameter at its tip of about 0.5 mm - 0.75 mm.

Absorbent material 26 may comprise any suitably absorbent material or synthetic fiber. Cellulose fibers are preferred.

In the illustrated embodiment, the absorbent material 26 of the invention is prepared in a manner similar to the preparation of an endodontic paper point. That is, a paper point is prepared by first forming a triangle of thin, absorptive parchment paper which is soaked in a solution of mucilage prepared from natural or synthetic polymers and is rolled diagonally to produce a cone that, when dried, is stiffened by the polymers so that it can be inserted into a root canal, cavity or periodontal pocket. After the mucilage is dried, a substrate which is reactive with a specific pathogen, as described herein, is impregnated on the surface of the cone. In an alternative embodiment, absorbent material 26 may be made by mixing a substrate with the mucilage.

Any substrate known in the art that is reactive with a particular biological material that is identifiable with oral disease can be used on a support used as described herein. Examples of such substrates used in the illustrated embodiment of the invention are:

1. N-benzoyl-D-L-arginine-2-napthylamide (BANA) which is reactive with trypsin to release $\beta$-napthylamide which is reactive with fast garnet to produce a bright red-orange color. (Preferred for extra oral use such as on support discs described below because of its mutagenic properties.)

2. benzoyl-arginine-4-methylcoumarin-7-amide and benzoyl-arginine-4-trifluoromethyl-coumarin-7-amide (AFC) which react with an antigen or antibody to produce a change in fluorescence. (4-methylcoumarin-7-amide and 4-trifluoromethyl-coumarin-7-amide are the preferred leaving groups for the substrates described herein regardless of whether the substrate is enzyme, antigen or antibody reactive.)

3. phenylphosphate and p-nitrophenyl-phosphate which react with phosphatase or transphosphorylating enzymes to release respectively, phenol, which is reactive with a diazo reagent to produce a sharp color change, and p-nitrophenol which undergoes a detectable color change on its own without a reagent.

4. Adenosine 5' - Triphosphate (ATP) is liberated from bacteria upon sonication. This sonicated extract, which may come from the gingival sulcus or the root canal space, is added to a standard reagent containing 0.02 M magnesium sulfate, luciferase, luciferin, DTT (1,4-Dithiothrietol), EDTA (Ethylene diamine tetraacetic acid), sodium salt, BSA (Bovine serum albumin), and a diluted tricine buffer. The presence of ATP from the sonicated bacterial extract produces a bioluminescent effect in the presence of these buffered reagents. This bioluminescence can be detected in a darkened room with the naked eye or, alternatively, with spectrophotometric devices.

Referring now to Figs. 3 and 4, the device 20 of the invention, or alternatively absorbent material 26 may be provided in a sterile package 40 which holds a number of devices 20 or absorbent material paper points (cones) 26. Package 40 has hinge 44 and snap connect 46 to provide for opening package 40 by moving top 43 on hinge 44 to separate top 43 from bottom 45. Package 40 has a number of bins 48 for receiving and holding devices 20 or cones 26. In the illustrated embodiment bins 48 are designed so that absorbent material 26 has minimal contact with the material used to construct box 40.

In its use, when measurements are desired, package 40 is opened (the package is stored in a dry condition) and each of devices 20 or cones 26 are removed from the package with handle 10 or forceps, and used to collect a fluid sample from the mouth of a patient. Then pad 42 is saturated with water and package 40 is closed and the package and its contents are incubated at a specific temperature for a time sufficient to cause a detectable reaction between the substrate and the biological fluid.

In the illustrated embodiment, device 20 or cones 26 are about 1 inch long. Package 40 is about 2-1/4 inches long, 1-3/4 inches wide and about 1/2 inch high. Pad 42 may comprise a swatch of tissue paper, gauze or a similar absorbent medium. In the illustrated embodiment package 40 is made of a clear plastic.

Those skilled in the art will recognize that in some instances the moisture on the paper point will provide sufficient humidity in the incubation step so that absorbent material and the addition of water to the package will not be required. Also, although illustrated as being oriented as being placed end to end between the hinge 44 and snap connector 46 in package 40, those skilled in the art will recognize that absorbent material 26 may be oriented in the package in any manner convenient for the practitioner. In a preferred embodiment a package (not illustrated) has been provided wherein the absorbent material 26 has been oriented substantially parallel to hinges 44 and snap connector 46.

Device 20 of the invention, made using AFC containing substrate for example, can be tested by inserting the absorbent material 26 portion into a 10 nmolar (10 nanomolar) solution of trypsin for a period of time sufficient to wet the absorbent material 26. After removing the absorbent material 26 from the solution, the device is covered to retain moisture for 10 minutes. The absorbed trypsin reacts with the AFC-containing substrate to release AFC. A drop of a solution of color developer, when touched to the base 32 of the point will react, after about 10 minutes of incubation, to cause a color change in the violet range of the spectrum as it reacts with the color developer, indicating that the trypsin has reacted and liberated AFC from the AFC containing substrate.

The same method can be used to test biological fluid in the mouth by inserting the absorbent material 26 into a root canal or a periodontal pocket to collect and measure the level of trypsin in the oral cavity, which can be correlated to proteolytic activity.

Similar procedures can be used when other substrates are used to detect other enzymes.

With reference now to Fig. 5, using information established herein (using specifically the support discs described below which have been treated in a manner similar to that described for the paper points), a color chart has been developed, having 13 shades of violet, which can be used to correlate concentrations of biological components to a particular shade of violet developed using a color indicator. The deeper shades of violet represent the higher concentrations of reactive biological material. Those skilled in the art will recognize that color charts using different colors can be developed depending on the color indicator used, and different ranges of colors may be developed depending on the substrates used. The particular color chart used in the illustrative embodiment is used for illustrative purposes only. Using the exemplary substrates and color indicators illustrated herein, it has been found that trypsin is the most sensitive biological component tested, and the illustrative color chart was developed using the colors generated by trypsin as a general guide.

In the actual use of the device of the invention on a patient, in for example the preparation of a root canal, the root canal may be debrided by an ultrasonic device while being simultaneously irrigated with an antibacterial solution of e.g. 2.5% sodium hypochlorite using a device known as the CAVITRON®/ENDOSONIC® endodontic tool, a product of Dentsply International. After debridement, the canal can be flushed with distilled water, and dried of excess water using an endodontic paper point. Absorbent material 26 can then be placed into the canal until fresh exudate of about 0.5 to 20, preferably 2 to 10 microliters volume is absorbed into the point. The device can then be removed and placed into a covered well to retain the moisture for about 10 to 30 minutes. If the device changes color to violet when touched with a drop of color developing solution, further debridement may be necessary. If there is no change in color, this indicates the absence of a measurable amount of proteolytic enzyme, and the cavity can then be filled.

In an alternative use of the device of the invention, after gaining access to the root canal space, for example, with rubber dam isolation, the practitioner may place an absorbent point into the canal space. Several points may be used if multiple tests are to be run. The practitioner may then remove the point from the canal space and place it directly into a tube containing a component (substrate or coupling agent) of a reagent system. The assistant or the practitioner may then add a second reagent (color developing reagent) or additional reagents and stir the tube manually. Color is determined over a predetermined period of time and read visually against a color guide or placed in a colorimeter to give a quantitative reading.

The presence of alkaline phosphatase indicates bone repair processes are at work. To illustrate a further embodiment of the present invention, the practitioner, after instrumentation is complete, places an appropriately sized paper point at or slightly beyond the apex of the tooth. The canal may be filled with sterile saline to facilitate diffusion of free tissue enzymes into the root canal space. The point is placed and removed with the placement instrument of the invention. The point is placed in a small vial of 1 to 2.5 mls of 1.5 mol/liter AMP buffer (2-Amino-2-methyl-1-propanol buffer), 1 to 2.5 mls of p-nitrophenol substrate in distilled water, 5 mg to 100 mgs per this volume. The reaction is allowed to proceed anywhere from 5 to 30 minutes. The yellow color is directly compared to an overlay color guide to allow assessment of the enzyme presence.

The same general procedure can be used to obtain fluid from a periodontal pocket and to analyze said fluid.

It will be recognized by those skilled in the art that other embodiments of the present invention can be used. For example, the device can be prepared by absorbing a color developer, as well as substrate on a paper support.

In an alternative embodiment of the device of the invention, the absorbent support material may be provided as part of a laminated composite. That is, one may coat the exterior of the absorbent material with an impermeable coating with an opening at the end 32 to selectively absorb fluid from the base of the cavity. Alternatively, there may be perforations along the length of the coating to provide areas of selective absorption. An example is a laminar composite in which the absorbent material is coated with a polymeric film and the end 32 cut away for absorption of fluid. Another example is a laminar composite that is made by placing a layer of non-absorbent material or membrane such as mylar over the absorbent paper prior to rolling the absorbent paper into a point. The absorbent paper and the non-absorbent material may be rolled together in a cone as previously described to produce the support used in the device of the invention. Those skilled in the art will recognize that other non-absorbent substances can be used to provide a laminated paper point.

The non-absorbent layer of material is provided to protect the absorbent material and prevent the washing away of substrate, for those substrates that may be susceptible to washing from the support by saliva. The non-absorbent layer may have perforations throughout the length of the device for permitting oral fluids to enter the absorbent paper through said perforations. Alternatively, the non-absorbent material may be provided without perforations, but may be open at the end 32 of device 20 to permit oral fluids to enter onto the absorbent paper by capillary action.

Such an alternative device may be used in the same manner described above.

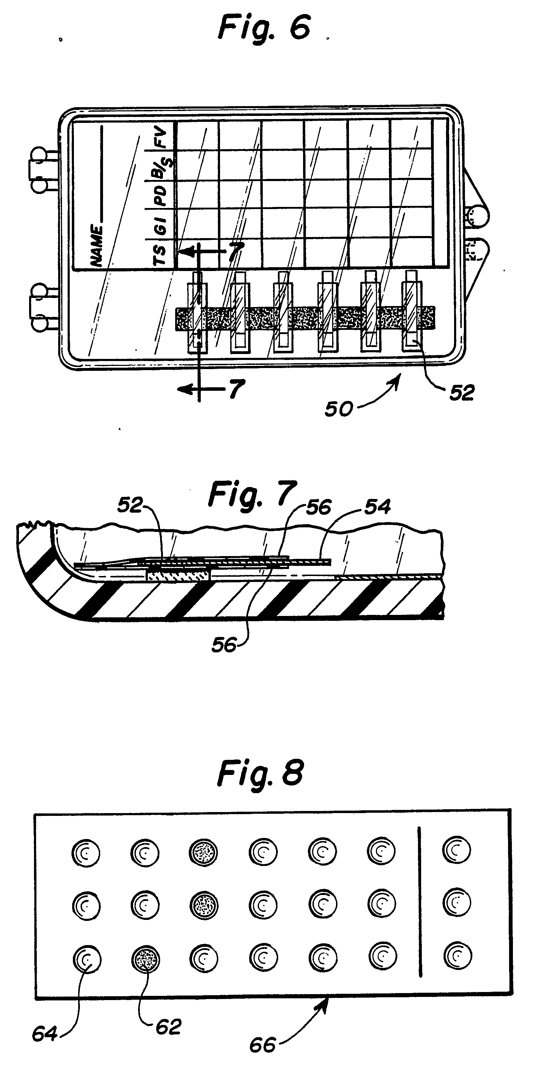

In an alternative embodiment, as illustrated in Figs. 6 and 7, instead of a paper point, a substrate impregnated paper strip may be used and packaged in an incubator box 50 similar to that described above. Such a paper strip 52 may be made by cutting filter paper 54, or similar absorbent material into strips 2 mm wide and sandwiching the paper 54 between two plastic portions 56 having a similar length and width so that 2 mm of the paper is exposed and available for absortion of the reactive biological fluid. The paper portion of the strip may be impregnated with substrate and the exposed paper 54 of strip 52 cut to size. (In the illustrated embodiment paper 54 is about 2 mm x 2 mm). In the preferred embodiment strip 52 will be about 8-10 mm long. The plastic portions 56 of the strip serve as means for picking-up and holding strips 52 in forceps. The length of strip 52 facilitates access of the strip in the posterior portions of the mouth. Also, plastic portions 56 are made having good flexibility such that the practitioner can flex or easily bend strip 52 for easier access to the mouth and so that chances of injury to the soft tissue of the sulcus are minimized while at the same time having sufficient rigidity such that manipulation of the strip can be easily controlled. Because of the small mass of absorbent material employed in strip 52 sampling of fluid (about 2 ul in the illustrated embodiment) in the gingival sulcus requires only about 2-30 seconds, and preferably 5-10 seconds.

Strips having a support of various dimensions up to about 4 mm x 4 mm, having various thicknesses, e.g. about 0.05 mm to about 1.0 mm may be provided. Accordingly, such strips may be used to obtain samples varying in volume from about 0.05 to about 10 ul, preferably about 0.5 to about 5 ul.

In one embodiment of such a paper strip, the substrate may be covalently bound to the paper.

Similar procedures as those described for testing a prepared root canal can be used for testing a periodontal pocket for the presence of proteolytic enzyme, antibody, or antigen activity.

Accordingly, the methods of making the device of the invention and for detecting oral disease comprises the steps of absorbing a substrate adapted to react chemically with biological materials that can be associated with oral disease onto an absorbent material which is dimensioned to fit into small recesses in the mouth, inserting the absorbent material with the absorbed substrate into a small recess in the mouth and absorbing biological material from the recess onto the absorbent material, allowing the biological material to react with the substrate for a set time sufficient for a measurable chemical reaction between the biological material and the substrate to take place, and measuring the extent of the chemical reaction to establish the concentration of the reactive component in the biological material. A number of specific substrates that are reactive with a number of specific biological materials may be used in the method of the invention. A preferred method is the detection of enzymes that are produced as a result of oral disease or diseases diagnosed through oral fluid.

Using a device such as strip 52, or a similar device, which has a predetermined area and absorptivity, makes possible the collection of a known volume of crevicular fluid, saliva or similar oral fluid. Accordingly, a known amount of said fluid can be collected, and in alternative embodiments, diagnosis can be obtained as described above using a substrate impregnated on the paper, or by eluting the known amount of collected fluid into a known amount of an appropriate buffer. Aliquots of the buffer and eluate can then be used to analyze the fluid on substrate impregnated paper discs in a procedure using the same chemical reactions and a similar procedure that is described for the paper points or paper strips above; or alternatively aliquots of the eluate can be incubated and reacted with an indicator and the color change measured in a colorimeter or fluorimeter to give an indication of the enzyme concentration; or in a further alterna tive embodiment, the aliquots can be used with a series of different substrates (on a support or in liquid form) to test for different viral infections such as human immuno deficiency (HIV) virus, hepatitis virus or for biological chemicals indicative of diabetes and for other diseases which may be of interest.

When no substrate is used on strip 52 it is used only as a collection means and for such purposes may be defined as a reservoir for crevicular fluid. For collection purposes, those skilled in the art will recognize that other suitable means, such as capillary tubes, may be used as a reservoir.

Other enzymes or factors of interest which can be analyzed according to alternative methods include but are not limited to: a) collagenase, b) acid phosphatase, c) beta glucuronidase, d) alpha Naphthyl Butyrate Esterase, e) proteases, f) lactate dehydrogenase (indicator of cell death and a necrotic pulp), g) arylsulfatase (indicator of inflammation), h) total protein, and i) urea.

Test of specific activity for substrate reaction to specific pathogens were carried out according to the following examples.

EXAMPLE 1

This example describes how substrates are incorporated into paper points and how paper points are used to test for the concentration of trypsin in a given solution.

Paper points were prepared, as described in the specification, by impregnating previously prepared and dried paper points by wetting the points in 0.25 mmolar (millimolar) Z-gly-gly-arg-AFC substrate (Lot #R108A-from Enzyme Systems Products [ESP]) Livermore, California. The specific Z blocking group used on the substrate was benzyloxycarbonyl. Medium sized absorbent paper points, available from The L. D. Caulk Company, a division of Dentsply International Inc., were used. It has been found that larger points will cause the sulcus to bleed, and smaller points do not have sufficient surface area to readily observe a color change. The substrate was prepared by dissolving 15.3 mg of the substrate in 1 ml DMF (N,N dimethylformamide) to make a 20 mmolar solution. The Dmf/substrate solution was then diluted 1:80 (q.s. to 80) in a buffer comprising 50 millimoles Tris (hydroxymethyl amino methane) HCL and 10 millimoles $CaCl_2$ in 1 liter of deionized water and having a pH of 8.0, to make a 0.25 mmolar solution of substrate.

The entire point was impregnated with the substrate and allowed to dry at room temperature overnight. The impregnated points were stored with a dessicant.

A 0.1 molar trypsin enzyme solution was prepared by dissolving 2.4 mg of trypsin (Sigma Chemical Co., St. Louis, Missouri) in 1 ml of the Tris-$CaCl_2$ buffer described above, and nine serial 1:10 dilutions of the original solution were made. Accordingly, 10 solutions having concentrations of 0.1 molar to 1 nanomolar (nmolar) of trypsin enzyme were tested.

One paper point, impregnated with Z-gly-gly-arg-AFC, was inserted into each of the trypsin solutions for a time sufficient to wet the paper point. Each of the paper points were then placed in a clear plastic case together with a water soaked absorbent material. The case and the paper points were then placed in an incubator at 37° C for 30 minutes. Immediately after completion of the incubation, each of the paper points were checked under ultraviolet light (preferably longwave, about 365 nm). It was found that low concentrations of trypsin cause the paper point to show light blue under the ultraviolet light, moderate concentrations of trypsin demonstrate visually detectable green-blue color to develop, higher concentrations of trypsin cause a green color to develop, and even higher concentrations of trypsin cause a bright green to develop. A zero concentration of trypsin is indicated by a deep blue color.

This information has been used to indicate whether a patient is positive or negative for the enzymes of interest.

This information can be used to develop a fluorescent color chart which can differentiate trypsin concentrations so that theoretically a quantitative representation of the infection can be obtained.

After the initial check under ultraviolet light, each of the paper points described above was contacted with a 0.057 molar solution of p-dimethylaminocinnamaldehyde (color developer) and incubated for 10 minutes. Each paper point in the series demonstrated a violet color which was distinguishable with the naked eye from each of the other paper points in the series. The paper points achieved a distinctively darker and visually discernable purple color as the concentration of trypsin increased.

The color developed on each of the paper points was compared with a color chart on which 13 distinctive shades of violet can be correlated with concentrations of trypsin in solution, as illustrated in Fig. 5 and described above.

It has been found that increasing the incubation temperature to 43° C gives a 5-10% reduction in reaction time of the trypsin with the substrate; the reaction can withstand temperatures up to about 80° C when a mammalian source trypsin sample is used.

Paper points have also been prepared using each of the substrates described in examples 3-7 and it has been found that the colors developed for specific concentrations of enzyme are the same as those developed on the paper discs of examples 2-7 below.

AFC is detected spectrophotometrically at about 380 nm, spectrofluorometrically by excitation at 400 nm and emission at 505 nm.

It has been found that blood interferes with or "blocks" the fluorescent reaction product and should be avoided to obtain an accurate result.

Clinically, a paper point impregnated with substrate is placed in the sulcus for 30 seconds and is then replaced into its plastic container. The paper point is then subjected to the test procedure described above.

EXAMPLE 1a

This example describes how the initial concentration of the enzyme standards used in example 1 and the following examples were prepared.

Preparation of enzyme solutions

Example

1. Trypsin enzyme
$$24{,}000 \text{ g/mole} \times 0.1 \text{ umole} \times \frac{1 \text{ mole}}{10^6 \text{ umole}} = 2.4 \text{ mg.}$$

2. Elastase enzyme
$$25{,}000 \text{ g/mole} \times 0.1 \text{ umole} \times \frac{1 \text{ mole}}{10^6 \text{ umole}} - 2.5 \text{ mg.}$$

3. B Collagenase
$$105{,}000 \text{ g/mole} \times 0.1 \text{ umole} \times \frac{1 \text{ mole}}{10^6 \text{ umole}} = 10.5 \text{ mg.}$$

Each enzyme was dissolved in 1 ml. of the appropriate buffer described in Table I to give a starting concentration of 100 umolar for Trypsin, Elastase, and Bacterial Collagenase, respectively.

4. DPPIV - The starting concentration comprising 0.19 umole solution is obtained commercially from ESP.

5. Cathepsin G - The starting concentration comprising 4.6 umolar solution is obtained commercially from Athens Research Technology, Athens, Georgia.

6. Cathepsin B - The starting concentration comprising 1 umolar solution is obtained commercially from ESP.

EXAMPLE 2

This example illustrates the preparation of paper discs, impregnated with substrate, that are used to quantify reactive species of biological materials outside the mouth when collected by other means.

With reference now to Fig. 8, discs of 3 mm chromatography paper 1/4 inch in diameter and 0.33 mm thick, were prepared by impregnating 5 x 5 inch pieces of paper by immersing the paper into 0.25 mmolar substrate solution (prepared according to Example 1) and allowing the paper to dry overnight by hanging the paper by one of its corners at room temperature and then by punching out discs 62 using a standard 1/4 inch paper punch. The diameter of the discs 62 is appropriate to be placed into the wells (depressions) 64 of a custom made test slide 66 made by Prototek (Dublin, CA).

One 1/4 inch disc was placed in each well of the test slide (which is set up for 6 clinical samples and 1 control for 3 different teeth) and wet with a drop of trypsin standard prepared according to Example 1. The discs were then incubated at 37°C for 30 minutes. The same procedures described in Example 1 were followed to observe the fluorescence and colors developed on the disc using the same color developer described in Example 1 yielding similar results.

## EXAMPLES 3-7

The procedures described in Example 2 were followed to test the reactivity of other pathogens, using substrates specific for those pathogens. Test data obtained for Examples 3-7 describe color properties developed using substrates specific for Elastase, Bacterial Collagenase, DPPIV, Cathepsin G and Cathepsin B, respectively. Described in the table below are the substrates used to test each enzyme, the source of the substrate, the buffer used as a carrier for each substrate, and the results obtained comparing the concentration of each enzyme tested with the color developed as compared to the color chart of Fig. 5. The numbers 1-13 at the right of the table relate directly to the shades indicated on the color chart.

Table I

| Example | ENZYME | SUBSTRATE | LOT # * | BUFFER | pH | INITIAL AMT SUBSTRATE ** | COLOR CHART CORRELATION WITH MOLAR CONCENTRATION OF ENZYME | | | | | | |
|---------|--------|-----------|---------|--------|-----|--------------------------|--------|--------|--------|--------|--------|--------|--------|
| | | | | | | | $10^{-4}$ | $10^{-5}$ | $10^{-6}$ | $10^{-7}$ | $10^{-8}$ | $10^{-9}$ | $10^{-10}$ |
| 2 | Trypsin-Like | Z-gly-gly-arg-AFC | R108A | TRIS | 8.0 | 15.3 mg/1 ml DMF | > 13 | 13 | 11 | 8 | 6 | 4 | |
| 3 | Elastase | MeoSuc-ala-ala-pro-Val-AFC | L-15 | HEPES | 7.5 | 11.5 mg/1 ml DMF | | | 11 | 8 | 6 | 2 | |
| 4 | Bacterial Collagenase | MeoSuc-gly-pro-leu-gly-pro-AFC | P-130 | TRIS | 7.5 | 15.3 mg/1 ml DMF | | | 6 | 3 | 1 | | |
| 5 | DPPIV | Z-Ala-pro-AFC | W-54 | 1.25% DMF | | 8.7 mg/1 ml DMF | See Table II | | | | | | |
| 6 | Cathepsin G | Suc-ala-ala-pro-phe-AFC | X057 | TRIS[1] | 7.5 | 14.3 mg/1 ml DMF | See Table II | | | | | | |
| 7 | Cathepsin B | Z-arg-arg-AFC | R-85B | DIT | 6.5 | 15.8 mg/1 ml DMF | | | 10 | 6 | < 1 | | |

*The source of each of the substrates is ESP

**125 ul of substrate/DMF solution is diluted q.s. to 10 ml with the appropriate buffer

Wherein DPPIV = dipeptidylaminopeptidase IV

AFC = 7-amino,4-trifluro methyl coumarin

Z = Benzyloxycarbonyl

MeoSuc = methoxysuccinyl

Suc = succinyl

DMF - N,N-dimethylformanide

TRIS = 50 mmoles Tris(hydroxymethyl)amino methane + 10 mmoles $CaCl_2$ in 1 liter deionized water

HEPES = 0.1 m hydroxyethyl piperazine-N-2 ethane sulfonic acid

TRIS[1] = 100 mmoles TRIS + 500 mmoles NaCl in 1 liter deionized water

DTT = 5 mmoles Dithiothreotol + 10 mmoles EDTA(sodium salt) + 50 mmoles $Na_3Po_4$ in 1 liter deionized water

Color developer = 1.0 gm (p-dimethylaminocinnamaldehyde) in 100 ml 10% H Cl

EP 0 304 871 A2

The Trypsin, Elastase and Bacterial Collagenase enzymes used for testing were prepared by dissolving 2.4 mg, 2.5 mg and 10.5 mg respectively of each enzyme in 1 ml of its appropriate buffer to give a starting concentration of 100 umolar in each case.

Cathepsin G is provided in solution in a concentration of 4.6 umolar from Athens Research Technology. Cathepsin B is provided in a umolar solution from ESP. DPPIV is provided in a 0.19 umolar solution from ESP.

Accordingly, the results obtained correlating the color chart with color developed by DPPIV and Cathepsin G are as follows.

TABLE II

| Concentration | Color Chart Numbers Correlation |
|---|---|
| DPPIV | |
| 0.19 umolar | 11 |
| 0.019 umolar | 8 |
| 0.0019 umolar | 4 |
| 0.00019 umolar | 0 |
| Cathepsin G | |
| 4.6 umolar | 11 |
| 0.46 umolar | 9 |
| 0.046 umolar | 6 |
| 4.6 nmolar | 3 |
| 0.46 nmolar | 1 |
| 0.046 nmolar | 0 |

EXAMPLE 8

This example illustrates a method for testing a known volume of crevicular fluid for biological activity so a calculation can be made to determine the concentration of the biological material in the crevicular fluid.

A periopaper gingival fluid collection strip (available from Interstate Drug Exchange, Amityville, N.Y. 11701-1130) was inserted into a periodontal pocket for about 3 minutes to absorb gingival crevicular fluid. The amount of fluid collected was measured by using a Periotron conductivity meter to measure the impedence of the sample. The collected gingival crevicular fluid was then eluted with an elution buffer (comprising 5 mmoles TRIS, 1 mmole NaCl, 1 mmole $CaCl_2$ and 1% (w/w) TRITON x-100 in 1 liter of deionized water, said buffer having a pH of 7.5) for 30-60 minutes at room temperature by placing the collection strip into 0.1 ml of the buffer. Aliquots of the eluate, 15 ul, were then placed onto substrate-impregnated discs (prepared as described in Example 2) which were previously placed in the wells of a Prototek test slide. Procedures of incubation and color development similar to those described in Examples 1-2 were then followed to determine the concentration of reactive biological material in the eluate.

The concentration of reactive biological species in the crevicular fluid was determined by observing the color developed on the chart, which indicates the concentration of the species in the aliquot, and calculating the concentration of oral fluid in the aliquot, and adjusting the concentration observed accordingly.

EXAMPLE 9

The following example illustrates the collection of precise volume of crevicular fluid on a substrate impregnated support. The same method has been used to collect a precise volume of fluid on a support made without substrate impregnation, and the fluid eluted in the same manner as described in Example 8.

Whatman #541 filter paper was sandwiched between two pieces of mylar having a width of about 6-8 mm. The exposed filter paper was then impregnated with substrate in the same manner described in Example 2. The dried substrate impregnated paper and plastic was then cut into 2 mm widths so that there was a 2 mm x 2 mm area of exposed paper at the end of each 2 mm x 8-10 mm strip.

Strips have also been made by cutting to size and impregnating the complete strip with substrate.

The strip was used to collect samples of fluid from the gingival sulcus by placing the paper portion of the strip into a perio pocket for 5-10 seconds picking up 2 ul of sulcular fluid. The paper was then processed as described in Example 1 to develop color indicative of the concentration of reactive enzymes in the sulcular fluid as described in Example 1.

EXAMPLE 10

In preliminary clinical tests, although specific correlation with eluted concentrations of bacteria could not be made in every case, a paper strip made according to Example 9, using a trypsin reactive substrate, was found to give accurate positive and negative results in patients (see Table III). Tests are continuing to establish a positive correlation between concentration and color development.

## TABLE III

| Patient # | Fluorescent Value | Total M/O $\times 10^7$ | Motile M/O $\times 10^7$ | Spirochetes $\times 10^7$ |
|---|---|---|---|---|
| 77 | | | | |
| Site 1 | 3 | 4.67 | 0.33 | 0.17 |
| Site 2 | 1 | 6.33 | 0.17 | 0.17 |
| Site 3 | 3B | 5.33 | 0.17 | 0.17 |
| 78 | | | | |
| Site 1 | 1 | 4.50 | 0.17 | 0.17 |
| Site 2 | 2 | 7.00 | 0.33 | 0.33 |
| Site 3 | 2 | 7.17 | 0.17 | 0.17 |
| 79 | | | | |
| Site 1 | 1B | 16.50 | 0 | 0 |
| Site 2 | 2B | 12.20 | 0 | 0 |
| Site 3 | 3 | 19.50 | 0 | 0.17 |
| 80 | | | | |
| Site 1 | 1B | 9.00 | 0 | 0.17 |
| Site 2 | 1B | 2.50 | 0.17 | 0.67 |
| Site 2 | 0B | 6.00 | 0.50 | 0 |
| 81 | | | | |
| Site 1 | 1 | 1.17 | 0 | 0 |
| Site 2 | 2B | 7.00 | 0 | 0 |
| Site 3 | 2B | 7.00 | 0.17 | 0 |
| 82 | | | | |
| Site 1 | 1 | 5.50 | 0.17 | 0 |
| Site 2 | 1B | 20.83 | 0 | 0 |
| Site 3 | 0B | 11.50 | 0 | 0 |
| 83 | | | | |
| Site 1 | 2 | 7.33 | 0 | 0 |
| Site 2 | 1 | 1.50 | 0.5 | 0 |
| Site 3 | 1 | 6.67 | 0 | 0 |

B in the fluorescent value indicates the presence of blood

M/O means microorganisms

The average quantity per field of total microorganisms, motile organisms and spirochetes were determined by viewing 6 fields of a Petroff-Hauser counting chamber under a dark-field microscope and dividing the number of microorganisms observed by 6. The fluorescent value is an empirical number between 0 and 4 obtained by observing various ranges of intensity of fluorescence developed by reacting various concentrations of trypsin with a substrate as described in Example 1.

While present embodiments of the invention and methods of practicing the same have been illustrated and described, it will be recognized by those skilled in the art that this invention may be otherwise variously embodied and practiced within the scope of the following claims.

## Claims

1. A device for detecting the presence of disease in the mouth comprising absorbent material having a dimension such that it can be inserted into a small recess in the mouth, wherein said absorbent material has an enzyme, antigen or antibody reactive substrate absorbed thereon which is adapted to react with organic biological materials that can be associated with disease.

15

2. The device according to Claim 1 which is adapted to be carried to the mouth by handling means in order to avoid contamination by human touch.

3. The device according to Claim 1 which has an indicator absorbed thereon which is adapted to react and change color when biological materials from the mouth react·with said substrate and release chemicals which indicate the presence of biological materials that can be identified with said disease.

4. The device according to Claim 1 in which said substrate changes color when a chemical reaction occurs between said substrate and biological materials that can be associated with said disease.

5. The device according to Claim 1 in which said substrate is covalently bound to said absorbent material.

6. A method of detecting the presence of disease in the mouth comprising the steps of

(a) inserting an absorbent material having an absorbed substrate thereon into a small recess in the mouth thereby absorbing biological material from said recess onto said absorbent material

(b) allowing said biological material to react with said substrate for a set time sufficient for a measurable chemical reaction between said biological material and said substrate to take place, and

(c) measuring the extent of said chemical reaction.

7. The method according to Claim 6 in which said recess is selected from the group comprising a root canal, a gingival sulcus, or other cavity in the mouth.

8. The method according to Claim 7 wherein said biological material is serum or exudate from a root canal, gingival sulcular fluid, whole saliva or other oral exudate.

9. The method according to Claim 8 in which the portion of said biological material reactive with said substrate is an enzyme, or antigen or an antibody.

10. The method according to Claim 6 comprising the step of selecting said substrate material from the group comprising a natural or synthetic enzyme, antibody or antigen.

11. The method according to Claim 9 comprising the step of indicating the extent of the reaction between the substrate and the reactive component of the biological material by measuring a change in color or fluorescence.

12. The method according to Claim 6 comprising the step of absorbing an indicator onto said absorbent material together with said substrate prior to collecting said biological material.

13. A method of making a device for detecting the presence of disease in the mouth comprising the steps of

(a) forming a support of absorbent material into a shape suitable for insertion into recesses in the mouth, and

(b) absorbing a chemical substrate on said support, said chemical substrate being adapted to react with biological materials in the mouth which are indicative of the presence of a diseased state in the mouth.

14. The method for making a device according to Claim 13 comprising the steps of providing said absorbent material in the form of a support made by forming a paper point by impregnating a triangle of paper with said substrate and rolling said triangle into a cone suitable for inserting into a recess in the mouth.

15. The method according to Claim 13 comprising the steps of using a synthetic enzyme reactive substrate on said support, and preparing said synthetic enzyme reactive substrate to have a leaving group that separates from said enzyme when said enzyme reacts with said reactive component of said biological material, and choosing said leaving group to be reactive with a specific color indicator, said reaction between the color indicator and the leaving group being adapted to cause a detectable and measurable color change so that the color change can be measured to indicate the concentration of the reactive component in the biological material.

16. The method according to Claim 15 wherein said leaving group comprises 4-methylcoumarin-7-amide.

17. The method according to Claim 15 wherein said leaving group comprises 4-trifluoromethylcoumarine-7-amide.

18. The method according to Claim 13 comprising the step of providing said absorbent material in the form of a support comprising a laminated composite of absorbent and non-absorbent layers, and separating absorbent layers containing said substrate from absorbent layers used to collect said biological material by a non-permeable membrane having perforations located to permit absorption of reactive components of the biological material from selected locations within the mouth.

19. The method according to Claim 13 comprising the step of choosing a substrate such that biological materials selected from the group consisting of antigenic components of the biological material, alkaline phosphate enzymes, proteolytic enzymes, and acid phosphate enzymes can be measured.

20. An apparatus for detecting state of disease in the mouth comprising a handle means and a state of disease detecting member having connecting means adapted to be held by said handle, in which said handle means has holding means associated therewith for releasable association with said connecting means of said state of disease detecting member whereby said handle is used for transporting said state of disease detecting member to the mouth to help prevent contamination.

21. The apparatus of Claim 20 wherein said disease state detecting member is a cone shaped point extending in a longitudinal direction and said connecting means is disposed perpendicular to said longitudinal direction.

22. The apparatus of Claim 20 wherein said disease state detecting member comprises a support having a substrate absorbed thereon.

23. The device according to Claim 1 which is adapted to pick up a specific amount of oral fluid.

24. A device for detecting the presence of disease in the mouth comprising a fluid reservoir sized to be completely filled with crevicular fluid upon being inserted into a gingival sulcus so as to contain a representative fluid aliquot of the normally existing fluid in said gingival sulcus.

25. The device of Claim 24 wherein said fluid reservoir is sized to contain from about 0.1 to about 10 ul gingival sulcus fluid.

26. The device of Claim 24 wherein said fluid reservoir comprises a strip of absorptive material sized to contain from about 0.05 to about 10 ul of gingival sulcus fluid when substantially fully impregnated.

27. The method of diagnosing disease in the mouth of a mammal comprising the steps of:

(a) inserting into a gingival sulcus a fluid reservoir sized to be completely filled upon being inserted into said gingival sulcus to collect a precise volume of crevicular fluid,

(b) retaining said fluid reservoir in said gingival sulcus for a time sufficient for complete filling of the reservoir but limited to a short enough time to substantially avoid new fluid from entering said gingival sulcus and filling said reservoir, and

(c) removing said filled fluid reservoir from said gingival sulcus before said time.

28. The method of Claim 27 comprising the further steps of

(a) eluting said precise volume of crevicular fluid in a precise volume of buffer

(b) transferring an aliquot of buffer containing said crevicular fluid to a substrate impregnated support in which said substrate is adapted to react with antigens, antibodies or enzymes identifiable with disease

(c) incubating said support and said aliquot for a time sufficient for a reaction between said substrate and said antigens, antibodies or enzymes to take place, and

(d) measuring the extent of said reaction to determine the concentration of antigen, antibodies or enzymes present.

29. The method of Claim 27 comprising the further steps of

(a) impregnating said fluid reservoir with an antibody, antigen or enzyme reactive substrate prior to inserting said reservoir into the gingival sulcus to collect crevicular fluid,

(b) incubating said reservoir containing substrate and crevicular fluid for a time sufficient for a reaction between said substrate and said antigens, antibodies or enzymes to take place, and

(c) measuring the extent of said reaction to determine the concentration of antigen, antibodies or enzymes present.

Fig. 1

Fig. 2

Fig. 3

Fig. 4

Fig. 5

## Fig. 6

## Fig. 7

## Fig. 8